## Europäisches Patentamt

## European Patent Office

## Office européen des brevets

(11) Veröffentlichungsnummer: **0 144 920**
**A2**

(12) **EUROPÄISCHE PATENTANMELDUNG**

(21) Anmeldenummer: **84114485.0**

(22) Anmeldetag: **29.11.84**

(51) Int. Cl.⁴: **A 61 N 1/42**

(30) Priorität: **08.12.83 DE 3344394**
**22.12.83 DE 3346401**

(43) Veröffentlichungstag der Anmeldung:
**19.06.85 Patentblatt 85/25**

(84) Benannte Vertragsstaaten:
**AT CH FR GB IT LI NL**

(71) Anmelder: **Schauf, G., Prof.**
**Parkstrasse 26**
**D-5600 Wuppertal 21(DE)**

(72) Erfinder: **Schauf, G., Prof.**
**Parkstrasse 26**
**D-5600 Wuppertal 21(DE)**

(74) Vertreter: **Patentanwälte Dr. Solf & Zapf**
**Schlossbleiche 20 Postfach 13 01 13**
**D-5600 Wuppertal 1(DE)**

(54) **Magnetfeld-Therapiegerät mit Vektor-Feldspulen.**

(57) Es wird ein Magnetfeld-Therapiegerät vorgeschlagen, mit dem ein Magnetfeld-Vektor erzeugbar ist, dessen Richtung und Intensität zeitlich variiert werden kann. Das Gerät umfaßt mindestens zwei Teilspulen (10a, 10b), denen mittels einer Ansteuerelektronik gleiche oder verschiedene Ströme zugeführt werden.

Fig. 4b

EP 0 144 920 A2

## Magnetfeld-Therapiegerät mit Vektor-Feldspulen

### Stand der Technik

Die Erfindung geht von einem Magnetfeld-Therapiegerät nach dem Oberbegriff des Patentanpruchs 1 aus.

In der medizinischen Therapie werden zunehmend niederfrequente Magnetfelder eingesetzt. Man verwendet hierzu Luftspulen, die ein magnetisches Feld erzeugen, das eine gleichbleibende Form hat, dabei jedoch mehr oder minder pulsiert. Die Felder werden in der Regel sinusförmig verändert, in einigen Fällen aber auch mittels Rechteckspannung oder anders geformter Signalspannungen beeinflußt. Die erzeugten elektromagnetischen Felder haben nachweislich in bestimmten Fällen positive therapeutische Wirkung, was auf eine Beeinflussung des Stoffwechsels zurückgeführt wird. Bei der Behandlung wird der Patient oder der zu behandelnde Körperteil in einen hohlzylindrischen Spulenkörper gebracht, in einigen Fällen auch direkt vor der Spule positioniert, und hier dem pulsierenden Feld der Spule ausgesetzt. Das Magnetfeld hat zur Folge, Magnetkräfte auf magnetisierbare Partikel und elektrische Kräfte auf elektrische Ladungsträger in stets gleichbleibender Richtung auszuüben. Die Intensitäten können dabei sowohl positive als auch negative Werte annehmen.

0144920

Bekannte Magnetfeld-Therapiegeräte arbeiten nur mit Feldern gleicher Richtung. Diese Geräte berücksichtigen also nicht, daß der Stoffwechsel im Körper nicht nur in einer Vorzugsrichtung, sondern stets in unterschiedlichen Richtungen verläuft.

Aufgabe der Erfindung

Der Erfindung liegt die Aufgabe zugrunde, mit einem Magnetfeld-Therapiegerät nach dem Oberbegriff des Anspruchs 1 ein möglichst räumlich definiertes Feld, homogen oder inhomogen (Gradientenfeld), zu erzeugen, daß in einem begrenzten Raumteil eine gezielte Therapie erlaubt. Weiterhin sollte das Magnetfeld gemäß einem wählbaren Programm seine räumliche Richtung ändern können. Dabei kann der Betrag des Feldes veränderlich oder auch konstant gewählt sein.

Vorteile der Erfindung

Das erfindungsgemäße Magnetfeld-Therapiegerät mit den kennzeichnenden Merkmalen des Anspruchs 1 hat den Vorteil, daß eine gezielte therapeutische Behandlung mit Vektorfeldern - auch pulsierend - von biologischen Substanzen möglich ist. In dem Behandlungsraum können je nach Größe der Spulenausführung sowohl Körperteile als auch ganze Lebewesen (Mensch, Tier oder Pflanze) eingebracht werden.

Durch die in den Unteransprüchen aufgeführten Maßnahmen sind vorteilhafte Weiterbildungen und Verbesserungen des in dem Anspruch 1 angegebenen Magnetfeld-Therapiegerätes möglich.

Beschreibung der Erfindung

Ausführungsbeispiele der Erfindung werden in der Zeichnung anhand mehrerer Figuren dargestellt und nachfolgend näher erläutert.

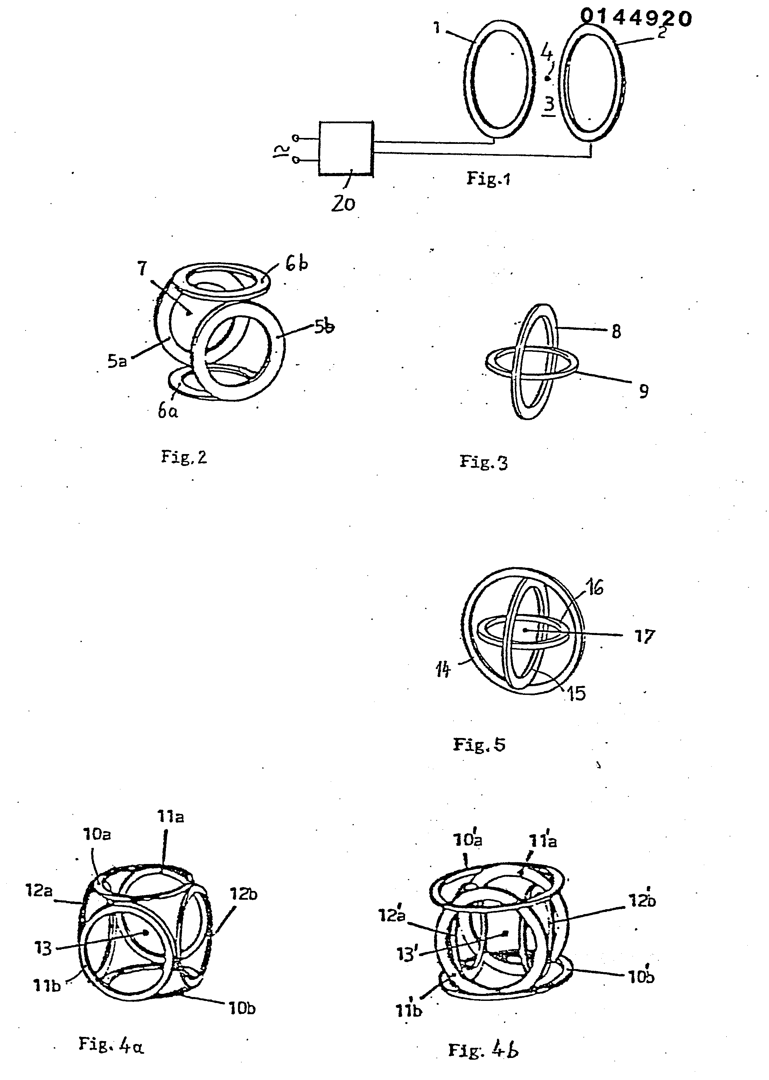

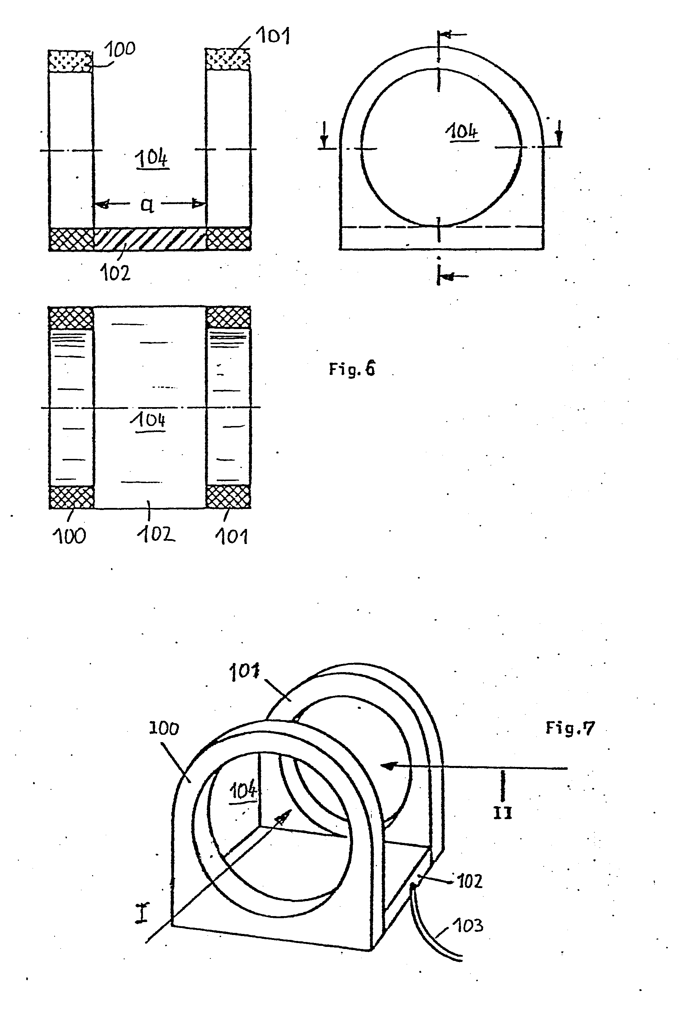

Fig. 1: Durch eine zeitlich unterschiedliche Ansteuerung zweier kurzer Teilspulen 1, 2 mit einer Ansteuerelektronik 20 kann in einem Behandlungsraum 3 das Überlagerungsfeld in einer Ebene seine Richtung ändern. Die Wirkung ist in einem mit 4 bezeichneten Punkt am stärksten; dieser Punkt ist also zur Behandlung mit einem rotierenden Vektor am besten geeignet. Durch entsprechende Ansteuerung der beiden Spulen kann außerdem im Behandlungsraum ein Magnetfeld mit definiertem Gradienten, also mit räumlicher Änderung, erzeugt werden, wenn der Spulenabstand a (vergleiche Fig. 6) gleichzeitig etwa dem halben Innendurchmesser der Spulen entspricht.

Fig. 2: Ordnet man zwei Spulenpaare 5a, 5b und 6a, 6b rechtwinklig zueinander an, wobei jedes Spulenpaar gemeinsam zur Erzeugung eines Feldvektors in einem Behandlungsraum 7 beiträgt, so ergibt sich je nach Ansteuerungsverhältnis der Spulen eine andere vektorielle Addition. Damit verbunden ist eine andere mögliche Wirkungsrichtung, die durch Phasensteuerung, also auch zeitlich variabel, beeinflußt werden kann.

Fig. 3: Setzt man zwei rechtwinklig verschachtelte Teilspulen 8, 9 ein, so erhält man ähnliche Verhältnisse wie bei der Ausführung nach Fig. 2. Dies gilt auch bei einer Mischausführung beider Lösungen.

Fig. 4a und 4b : Setzt man drei Spulenpaare 10a, 10b; 11a, 11b; 12a, 12b; (Fig. 4a) beziehungsweise 10´a, 10´b; 11´a, 11´b; 12´a, 12´b; (Fig. 4b) ein, so ist die Erzeugung von Feldvektoren beliebiger Richtung unterschiedlicher Intensität im Behandlungsraum 13, 13´ möglich. Die Spulenpaare gemäß den Fig. 4a und 4b unterscheiden sich dadurch, daß nach Fig. 4a Spulen gleichen Durchmessers Verwendung finden, während nach Fig. 4b die Spulen des Spulenpaares 10´a, 10´b einen größeren Innendurchmesser haben als der Außendurchmesser der Spulen des Spulenpaars 11´a, 11´b und daß der Innendurchmesser der Spulen des Spulenpaars 11´a und 11´b größer ist als der Außendurchmesser der Spulen des Spulenpaars 12´a, 12´b.

Fig. 5: Nach Fig. 5 ist eine Vereinfachung der Lösung nach Fig. 4 in Stufen möglich. Fig. 5 zeigt die größtmögliche Vereinfachung mit nur drei Teilspulen 14, 15, 16. Ein Überlagerungseffekt durch die Felder der einzelnen Spulen ist ebenfalls gegeben; in dem Behandlungsraum 17 treten jedoch keine homogenen Feldverhältnisse auf.

Fig. 6 und 7: Nach den Fig. 6 und 7 sind zwei Teilspulen 100, 101 lösbar über einen, plattenförmigen Zwischenkörper 102 verbunden. Die Teilspulen sind vorzugsweise in Isolierstoff gehaltert; sie weisen einen Abstand a auf. Bei dieser Anordnung kann das zu behandelnde Objekt sowohl in Achsrichtung der Spulen (Pfeilrichtung I) als auch quer zu dieser Achsrichtung (vergleiche Pfeilrichtung II) in den Behandlungsraum 104 eingeführt werden. Die zur Erzeugung der Magnetfelder benötigten Ströme werden über Anschlußdrähte 103, die vorzugsweise innerhalb des Zwischenkörpers 102 verlaufen, den Spulen 100, 101 zugeführt. Die Durchmesser der Teilspulen können verschieden groß gewählt werden, um das resultierende Feld zu beeinflussen. Durch die lösbare Verbindung zwischen dem Zwischenkörper 102 und den Teilspulen 100, 101 können die Teilspulen auch einzeln zu Therapiezwecken verwendet werden.

Patentansprüche

1. Magnetfeld-Therapiegerät, bestehend aus einer Ansteuerelektronik und einem damit verbundenen Spulensystem, dadurch gekennzeichnet, daß das Spulensystem (Fig. 1,...) aus mindestens zwei Teilspulen (1, 2) besteht, die durch die Ansteuerelektronik (20) getrennt ansteuerbar sind.

2. Gerät nach Anspruch 1, dadurch gekennzeichnet, daß die Teilspulen (1, 2) derart ansteuerbar sind, daß im Behandlungsbereich (3) resultierende Feldvektoren erzeugt werden, die unterschiedliche Richtung und Intensität oder unterschiedliche Gradientenwerte haben.

3. Gerät nach Anspruch1, dadurch gekennzeichnet, daß bei zwei oder mehreren Teilspulen oder Spulenpaaren (5a, 5b; 6a, 6b) die einzelnen Spulen zeitmäßig und strommäßig derart ansteuerbar sind, daß im Behandlungsraum die Richtung, die Intensität und der Gradient des Feldes gezielt einstellbar und/oder veränderbar sind.

4. Gerät nach Anspruch 1, dadurch gekennzeichnet, daß die Spulen (100, 101) in zwei lösbar miteinander verbundenen Teilkörpern untergebracht sind, so daß wahlweise ein Behandlungsgegenstand axial (Pfeilrichtung I in Fig. 7) oder radial (Pfeilrichtung II) in den Behandlungsraum (104) einbringbar ist.

5. Gerät nach Anspruch 2, dadurch gekennzeichnet, daß die Spulen (100, 101) in definiertem Abstand (a) fixiert werden.

6. Gerät nach Anspruch 2, dadurch gekennzeichnet, daß die Spulen (100, 101) so ausgeführt sind, daß sie einzeln zur inhomogenen Behandlung geeignet sind.

7. Gerät nach einem der Ansprüche 1 bis 3, dadurch gekennzeichnet, daß die Spulen der Spulenpaare (10´a, 10´b; 11´a, 11´b) verschiedene Durchmesser aufweisen (Fig. 4b) und ineinander geschachtelt sind.

8. Gerät nach einem der Ansprüche 4 bis 6, dadurch gekennzeichnet, daß die Teilspulen (100, 101) unterschiedliche Durchmesser aufweisen.

9. Gerät nach einem der Ansprüche 1 bis 8, dadurch gekennzeichnet, daß die Spulen als Kurzspulen ausgebildet sind.

10. Gerät nach einem der Ansprüche 4 bis 6, dadurch gekennzeichnet, daß der Abstand (a) zwischen den Teilspulen (100, 101) etwa dem halben Innendurchmesser der Teilspulen entspricht.

0144920

Fig.1

Fig.2

Fig.3

Fig.5

Fig.4a

Fig.4b

Fig. 6

Fig. 7